Europäisches Patentamt

European Patent Office

Office européen des brevets

(19)

(11) **EP 1 508 362 A1**

(12) **DEMANDE DE BREVET EUROPEEN**

(43) Date de publication:
23.02.2005 Bulletin 2005/08

(51) Int Cl.$^7$: **B01D 65/10**, G01N 15/08, A61L 2/28

(21) Numéro de dépôt: 04292042.1

(22) Date de dépôt: 12.08.2004

(84) Etats contractants désignés:
AT BE BG CH CY CZ DE DK EE ES FI FR GB GR HU IE IT LI LU MC NL PL PT RO SE SI SK TR
Etats d'extension désignés:
AL HR LT LV MK

(30) Priorité: 19.08.2003 FR 0350436

(71) Demandeur: L'air liquide - Société anonyme à Directoire et Conseil de Surveillance pour l'Etude et l'Exploitation des Procédés G. Claude
75321 Paris Cedex 07 (FR)

(72) Inventeurs:
• Dupont, Pascale
92340 Bourg la Reine (FR)

• Girault, Christel
78960 Voisins le Bretonneux (FR)
• Germain, Jean Pierre
78180 Montigny le Bretonneux (FR)
• Gouy-Pailler, Philippe
75001 Paris (FR)

(74) Mandataire: Mellul-Bendelac, Sylvie et al
L'Air Liquide,
Service Propriété Intellectuelle,
75, Quai d'Orsay
75321 Paris Cedex 07 (FR)

(54) **Procédé et installation de validation d'un filtre stérilisant ou d'abattement microbien en conditions d'utilisation**

(57) Un procédé de validation d'un filtre stérilisant en conditions d'utilisation qui comporte une étape de vérification de l'efficacité stérilisante du filtre dans les conditions d'utilisation par un test de challenge microbien ou particulaire, réalisé avec le produit à filtrer et à la température du produit à filtrer, selon lequel on abaisse la température du filtre jusqu'à la température du produit à filtrer et on le maintient à cette température pendant toute la durée de la procédure, on injecte dans le filtre une quantité déterminée du produit à filtrer, préalablement chargé en microorganismes ou en particules selon une concentration prédéfinie, et on vérifie que le filtrat est stérile, le produit à filtrer ayant été préalablement chargé en microorganismes ou en particules selon ladite concentration prédéfinie par l'utilisation d'un réservoir intermédiaire destiné à stocker du produit à filtrer dans lequel on a introduit un inoculum microbien ou particulaire en solution, on a laissé l'inoculum ainsi introduit s'évaporer, on a abaissé la température du réservoir jusqu'à la température du produit à filtrer et on le maintient à cette température , on verse du produit à filtrer dans le réservoir ainsi contaminé, et on vérifie en sortie du réservoir que le produit à filtrer contient une quantité de microorganismes ou de particules conforme aux spécifications recherchées.

**Description**

La présente invention concerne le domaine des filtres stérilisants ou dits « d'abattement microbien ». Elle s'intéresse plus particulièrement au cas de la filtration des liquides cryogéniques.

En considérant dans ce qui suit l'exemple des filtres dits « stérilisants », ceux-ci doivent être l'objet de tests destinés à prouver leur efficacité stérilisante.

Deux types de tests sont couramment utilisés :

- Le « challenge bactérien » est un test généralement destructif dont la méthodologie est bien connue, et qui permet de donner à une référence de filtres l'appellation « filtre stérilisant »,
- Le « test d'intégrité » est un test non destructif, qui fait l'objet d'une corrélation avec le challenge bactérien, et qui permet ainsi de vérifier l'efficacité du filtre au cours de sa période d'utilisation. Il est typiquement réalisé par Test de Point de Bulle, Test de Diffusion, ou Test d'Intrusion d'Eau.

Le challenge bactérien est généralement réalisé au stade laboratoire par le fabricant du filtre. Son objectif est de prouver l'efficacité stérilisante d'une référence de filtres, pour pouvoir lui donner l'appellation « filtre stérilisant ».

La méthodologie du challenge bactérien, dans des conditions standards, est typiquement la suivante :

- Toutes les étapes sont réalisées à température ambiante.
- Une solution bactérienne aqueuse à une concentration de $10^7$ bactéries par $cm^2$ de surface filtrante est préparée puis nébulisée (aérosol) ou injectée (liquide) dans le filtre selon un protocole connu (par exemple selon la norme ASTM F838-83 ou encore Guideline N°19 du EHEDG).
- Le filtrat doit être stérile pour que le filtre puisse être considéré comme « stérilisant » (voir les Guideline on sterile drug products produced by aseptic processing, June 1987, US F.D.A).

Compte-tenu de la méthodologie, ce test peut être destructif par le fait que la méthode contamine le filtre de façon massive et irréversible et ne peut donc pas être réalisée sur les filtres qui sont destinés à être utilisés. Elle n'est donc notamment pas compatible avec une utilisation en ligne.

Venons en maintenant au test d'intégrité qui permet quant à lui de tester de façon non destructive les filtres qui sont utilisés. Il est corrélé au challenge bactérien par des essais en laboratoire selon les règles de l'art. Il suffit donc ensuite, lors de son utilisation, de vérifier que le test d'intégrité d'un filtre est correct pour prouver son efficacité.

Le test d'intégrité est couramment employé dans l'industrie pharmaceutique, il peut être réalisé en ligne, ou au laboratoire. Il est généralement effectué sur le filtre avant sa mise en service et à la fin de son utilisation, pour s'assurer que pendant toute sa période d' utilisation le filtre était efficace. Sur des procédés sensibles, le test d'intégrité peut même être réalisé après chaque cycle de stérilisation, avant et après chaque cycle de production ou encore après chaque lot de fabrication.

Une des méthodologies de test d'intégrité pratiquée dans des conditions standards est la suivante :

- Toutes les étapes sont réalisées à température ambiante.
- Les pores du filtre sont saturés avec une solution aqueuse, puis il est injecté dans le filtre un débit constant et contrôlé d'air comprimé, la pression de l'air comprimé est augmentée graduellement jusqu'à obtenir l'expulsion de la solution aqueuse des pores du filtre.

Cette pression d'expulsion est différente d'un type de filtre à l'autre mais elle est déterminée par le fabricant du filtre. Le test vérifie donc que le filtre en place a bien des caractéristiques comparables à celles données par le fabricant.

Or dans certains cas, le produit à filtrer n'est pas compatible avec un test en phase aqueuse (par exemple pour des raisons de température, ou encore dans le cas de fluides liquides hydrophobes tels des huiles, ou encore dans le cas d'utilisation de filtres hydrophobes). Un mouillage du filtre avec une solution aqueuse pour réaliser le test d'intégrité ne permet pas de filtrer le produit immédiatement après la réalisation du test. Une étape de séchage est alors nécessaire.

Un des objectifs de la présente invention est alors de proposer des conditions de test permettant d'éviter la nécessité d'un séchage du filtre après réalisation du test.

Mais d'autre part, il faut noter que les tests aqueux à température ambiante ne sont pas les plus adaptés à la validation d'un filtre stérilisant utilisé en conditions non aqueuses et à température non ambiante, comme c'est le cas lorsque l'on veut filtrer des fluides cryogéniques.

Il semble alors nécessaire de pouvoir proposer une méthodologie de test la plus proche possible des conditions d'utilisation du filtre, y compris donc en conditions cryogéniques.

Il faut souligner ici l'approche du document DE-33 42 440 qui propose un test d'intégrité de filtres sur des liquides cryogéniques (point de bulle : mouillage du filtre à l'azote liquide, injection d'hélium gazeux et mesure de la diffusion de l'hélium dans le filtre). Pour déterminer la valeur limite du test d'intégrité pour laquelle le filtre est considéré comme intègre, l'auteur détermine cette valeur limite de façon théorique, en pondérant le point de bulle qu'il a déterminé expérimentalement par la valeur limite annoncée par le fournisseur pour un test d'intégrité à l'eau, en fonction de la tension superficielle de l'azote liquide par rapport à celle de l'eau. Cette valeur limite reste alors théorique et de plus ne tient pas compte des propriétés du gaz propulseur (i.e l'hélium par rapport à celles de l'air). D'autre part, le test d'intégrité ainsi réalisé en conditions cryogéniques n'est pas corrélé à un challenge bactérien, et ne parait donc pas suffisant pour valider l'efficacité micro-biologique du filtre.

On pourra aussi se reporter au document paru dans Journal of Pharmaceutical Science and Technology, 1998 supplement volume 52 number S1, Page 15, qui évoque l'utilisation du produit à filtrer comme fluide de mouillage du filtre pour les tests d'intégrité, et la corrélation qu'il effectue de façon expérimentale entre les valeurs obtenues durant le test d'intégrité effectué à l'aide du produit à filtrer, avec celles obtenues de façon standard par le constructeur par le test à l'eau.

Selon la présente invention, on propose une nouvelle méthodologie de test de filtres stérilisants ou dits « d'abattement microbien » comprenant les étapes suivantes :

*Etape a)* : la validation du filtre stérilisant dans les conditions standards, selon les règles de l'art (challenge bactérien et test d'intégrité réalisés à température ambiante avec des solutions aqueuses) ;

*Etape b)* : la réalisation d'un test d'intégrité dans les conditions d'utilisation i.e avec le produit à filtrer (et non de l'eau) et à la température du produit à filtrer, ceci selon la méthodologie suivante :

- Abaisser la température du filtre jusqu'à la température du produit à filtrer et le maintenir à cette température pendant toute la durée du test,
- Saturer les pores du filtre avec le produit à filtrer et injecter un gaz propulseur qui reste à l'état gazeux à la température du produit à filtrer,
- Augmenter graduellement la pression du gaz propulseur jusqu'à obtenir l'expulsion du produit à filtrer des pores du filtre.
  La pression d'expulsion ainsi obtenue dépend de la mouillabilité du produit à filtrer et doit être comparée aux caractéristiques données par le fabricant. Pour ce faire, une correspondance est établie entre le test d'intégrité réalisé dans les conditions standards et le test d'intégrité réalisé dans les conditions d'utilisation, de façon à déterminer la valeur limite de façon expérimentale, en tenant compte du produit, du gaz propulseur, et des conditions réelles d'utilisation.
  Pour cela on utilise la formule classique :

Valeur limite du PdB dans le produit =

valeur limite du PdB dans eau x (PdB moyen dans produit / PdB

moyen dans eau)

où l'abréviation PdB désigne le Point de Bulle.
Le test d'intégrité décrit dans cette étape b) est donc un moyen de s'assurer en ligne de l'efficacité du filtre. Par ailleurs l'observation même de la courbe d'intégrité fournit une information sur le fait que le filtre n'est pas endommagé.

*Etape c)* : Vérification de l'efficacité stérilisante du filtre dans les conditions d'utilisation par un test de challenge microbien ou particulaire, réalisé avec le produit à filtrer, et non de l'eau, et à la température du produit à filtrer, selon la méthodologie suivante :

- Abaisser la température du filtre jusqu'à la température du produit à filtrer et le maintenir à cette température pendant toute la durée du test (par exemple, par immersion du filtre dans un bain de liquide cryogénique ou par contact direct avec le liquide cryogénique) ;
- Injecter dans le filtre le produit à filtrer, préalablement chargé en microorganismes ou en particules ;
- Vérifier que le filtrat est stérile.

La difficulté réside ici, on l'aura compris, dans la contamination du produit à filtrer.

Dans le cas d'un challenge microbien, les solutions microbiennes sont habituellement des solutions aqueuses, qui ne seront souvent pas compatibles avec le produit à filtrer, par exemple avec un liquide cryogénique.

La méthodologie de contamination du produit à filtrer proposée ici est alors la suivante :

- Verser un inoculum microbien (en solution, aqueuse ou partiellement éthylique ce qui permet une évaporation ultérieure facilitée) dans un réservoir destiné à stocker le produit à filtrer. Ce réservoir aura été nettoyé, désinfecté et séché au préalable (selon des méthodes communément pratiquées par l'homme de métier). L'inoculum microbien aura été préparé de façon à résister à la température du produit à filtrer (par exemple une température cryogénique ; on utilisera alors préférentiellement des souches résistantes aux variations de température, par exemple des spores de *Bacillus subtilis).* Sa concentration aura été déterminée préférentiellement de façon à obtenir au final un produit à filtrer contaminé selon les spécifications d'un challenge bactérien standard.
- Laisser l'inoculum microbien s'évaporer le temps nécessaire.
- Abaisser la température du réservoir jusqu'à la température du produit à filtrer et le maintenir à cette température.
- Verser le produit à filtrer (par exemple le liquide cryogénique) dans le réservoir ainsi contaminé.
- Vérifier en sortie du réservoir que le produit à filtrer contient une quantité de microorganismes conforme aux spécifications requises, par exemple celles d'un challenge bactérien standard (par exemple en utilisant un système mettant en série un évaporateur et un biocollecteur par impaction).

On peut, par ailleurs, réaliser un challenge particulaire pour pallier la difficulté de contamination microbienne d'un produit. Dans ce cas, on contamine le produit à filtrer avec des particules ayant, à la température de filtration, une taille appropriée aux caractéristiques du filtre à valider (taille des pores notamment). On peut par exemple utiliser des particules de silice.

La méthodologie en trois étapes décrite ci-dessus permet de valider l'efficacité stérilisante du filtre, pour le produit à filtrer, et dans les conditions de température de filtration, et de déterminer sa valeur d'intégrité en conditions cryogéniques.

Le test d'intégrité réalisé dans les conditions d'utilisation, décrit ci-dessus, peut être réalisé en ligne, de façon régulière, très aisément, puisqu'il ne nécessite pas d'être suivi par un séchage.

En résumé, par rapport à l'approche du document Allemand cité ci-dessus, le test d'intégrité est réalisé en condition d'utilisation et corrélé au challenge bactérien réalisé en conditions d'utilisation (et non comme proposé par cet art antérieur une corrélation au challenge bactérien aqueux, ce qui n'est pas satisfaisant car éloigné des conditions réelles d'utilisation).

Dans tout ce qui précède et ce qui suit concernant la présente invention, la notion de « filtre » doit s'entendre comme recouvrant aussi bien le media filtrant seul que l'ensemble media filtrant dans sa cartouche.

En conséquence, le procédé de validation d'un filtre stérilisant ou d'abattement microbien en conditions d'utilisation selon l'invention se caractérise en ce qu'il comporte une étape de vérification de l'efficacité stérilisante du filtre dans les conditions d'utilisation par un test de challenge microbien ou particulaire, réalisé avec le produit à filtrer et à la température du produit à filtrer, selon la procédure suivante :

- on abaisse la température du filtre jusqu'à la température du produit à filtrer et on le maintient à cette température pendant toute la durée de la procédure ;
- on injecte dans le filtre une quantité déterminée du produit à filtrer, préalablement chargé en microorganismes ou en particules selon une concentration prédéfinie ;
- on vérifie que le filtrat est stérile ;

le produit à filtrer ayant été préalablement chargé en microorganismes ou en particules selon ladite concentration prédéfinie, de la façon suivante :

i) on dispose d'un réservoir intermédiaire destiné à stocker du produit à filtrer ;
j) on introduit un inoculum microbien ou particulaire en solution dans ledit réservoir et on laisse l'inoculum ainsi introduit s'évaporer ;
k) on abaisse la température du réservoir jusqu'à la température du produit à filtrer et on le maintient à cette température ;
l) on verse du produit à filtrer dans le réservoir ainsi contaminé ;
m) on vérifie en sortie du réservoir que le produit à filtrer contient une quantité de microorganismes ou de particules conforme aux spécifications recherchées.

Comme il apparaîtra clairement à l'homme du métier, quand on dit ci-dessus que « on laisse l'inoculum ainsi introduit s'évaporer », on doit comprendre que l'on laisse évaporer naturellement ou en aidant quelque peu par un apport de chaleur mais toujours dans des conditions maintenant la viabilité des microorganismes introduits.

Le procédé pourra par ailleurs adopter l'une ou plusieurs des caractéristiques suivantes :

- ledit abaissement de la température du filtre ou du réservoir est effectué par immersion du filtre ou du réservoir dans un bain de liquide cryogénique ou par contact direct avec un liquide cryogénique.
- le produit à filtrer obtenu en sortie de réservoir contient une quantité de microorganismes ou de particules permettant d'obtenir au final un produit à filtrer contaminé selon les spécifications d'un challenge bactérien standard.
- le procédé comporte au préalable une étape de validation du filtre stérilisant dans des conditions standards selon les règles de l'art i.e comportant un challenge bactérien et un test d'intégrité réalisés à température ambiante avec des solutions aqueuses.
- ladite étape de validation dans des conditions standards est suivie d'une étape de réalisation d'un test d'intégrité dans les conditions d'utilisation du filtre i.e avec le produit à filtrer et à la température du produit à filtrer, selon la méthodologie suivante :

  i) on abaisse la température du filtre jusqu'à la température du produit à filtrer et on le maintient à cette température pendant toute la durée de ladite étape de validation dans des conditions standards,
  j) on sature les pores du filtre avec le produit à filtrer,
  k) on procède à l'injection d'un gaz propulseur qui reste à l'état gazeux à la température du produit à filtrer,
  l) on augmente graduellement la pression du gaz propulseur jusqu'à obtenir l'expulsion du produit à filtrer des pores du filtre et obtenir ainsi une valeur de pression d'expulsion.

- on effectue une corrélation entre le test d'intégrité ainsi réalisé dans les conditions d'utilisation avec le test d'intégrité réalisé dans les conditions standards de façon à déterminer la valeur limite de pression, en tenant compte de la nature du produit, de la nature du gaz propulseur, et desdites conditions d'utilisation.
- l'observation même de l'allure de la courbe d'évolution de la pression permet de contrôler que le filtre n'est pas endommagé.
- on effectue une corrélation entre ledit test d'intégrité réalisé dans les conditions d'utilisation et ledit test de challenge microbien ou particulaire réalisé avec le produit à filtrer.

Une telle corrélation peut se faire par une procédure typiquement utilisée par les fournisseurs de filtre, à savoir : un panel de filtres est testé, tout d'abord par test d'intégrité, puis par challenge bactérien. Sur ce panel de filtres, certains ne "passent" pas le test de challenge bactérien, c'est-à-dire donnent lieu à un effluent qui n'est pas stérile. La valeur de diffusion ou de point de bulle limite est la valeur permettant d'éliminer tous les filtres qui n'ont pas passé le test de challenge bactérien (avec une marge de sécurité).

Comme on l'aura compris à la lecture de ce qui précède, la corrélation entre le test d'intégrité ainsi réalisé dans les conditions d'utilisation avec le test d'intégrité réalisé dans les conditions standards fournit une première valeur limite de pression.

La corrélation entre le test d'intégrité réalisé dans les conditions d'utilisation et le test de challenge microbien ou particulaire réalisé avec le produit à filtrer fournit quant à elle une seconde valeur limite de pression.

On prendra alors avantageusement on le conçoit la valeur la plus sécuritaire

En deçà de cette valeur de pression la plus sécuritaire, les filtres sont généralement considérés comme non intègres.

La présente invention concerne également une installation de validation d'un filtre stérilisant ou d'abattement microbien, en conditions d'utilisation, caractérisée en ce qu'elle comporte :

- des moyens pour abaisser la température du filtre jusqu'à la température du produit à filtrer et de maintenir le filtre à cette température pendant toute la durée de la procédure ;
- des moyens pour injecter dans le filtre une quantité déterminée du produit à filtrer, préalablement chargé en microorganismes ou en particules selon une concentration prédéfinie ;

  lesdits moyens pour injecter dans le filtre une quantité déterminée du produit à filtrer, préalablement chargé en microorganismes ou en particules selon une concentration prédéfinie comprenant :

  i) un réservoir intermédiaire destiné à stocker du produit à filtrer ;
  j) un inoculum microbien ou particulaire en solution ;
  k) des moyens d'introduction dudit inoculum microbien ou particulaire en solution dans ledit réservoir et d'évaporation dudit inoculum ainsi introduit.
  l) des moyens d'abaissement de la température du réservoir jusqu'à la température du produit à filtrer et de maintien de la température du réservoir à cette température ;

m) des moyens d'introduction du produit à filtrer dans le réservoir ainsi contaminé après évaporation dudit inoculum ;

la concentration microbienne ou particulaire dudit inoculum en solution étant apte à permettre que le produit à filtrer obtenu en sortie du réservoir contienne une quantité de microorganismes ou de particules conforme aux spécifications recherchées.

**Revendications**

1. Procédé de validation d'un filtre stérilisant ou d'abattement microbien en conditions d'utilisation **caractérisé en ce qu'**il comporte une étape de vérification de l'efficacité stérilisante du filtre dans les conditions d'utilisation par un test de challenge microbien ou particulaire, réalisé avec le produit à filtrer et à la température du produit à filtrer, selon la procédure suivante :

   - on abaisse la température du filtre jusqu'à la température du produit à filtrer et on le maintient à cette température pendant toute la durée de la procédure ;
   - on injecte dans le filtre une quantité déterminée du produit à filtrer, préalablement chargé en microorganismes ou en particules selon une concentration prédéfinie ;
   - on vérifie que le filtrat est stérile ;

   le produit à filtrer ayant été préalablement chargé en microorganismes ou en particules selon ladite concentration prédéfinie, de la façon suivante :

   i) on dispose d'un réservoir intermédiaire destiné à stocker du produit à filtrer ;
   j) on introduit un inoculum microbien ou particulaire en solution dans ledit réservoir et on laisse l'inoculum ainsi introduit s'évaporer ;
   k) on abaisse la température du réservoir jusqu'à la température du produit à filtrer et on le maintient à cette température ;
   l) on verse du produit à filtrer dans le réservoir ainsi contaminé ;
   m) on vérifie en sortie du réservoir que le produit à filtrer contient une quantité de microorganismes ou de particules conforme aux spécifications recherchées.

2. Procédé de validation selon la revendication 1, **caractérisé en ce que** ledit abaissement de la température du filtre ou du réservoir est effectué par immersion du filtre ou du réservoir dans un bain de liquide cryogénique ou par contact direct avec un liquide cryogénique.

3. Procédé de validation selon la revendication 1 ou 2, **caractérisé en ce que** le produit à filtrer obtenu en sortie de réservoir contient une quantité de microorganismes ou de particules permettant d'obtenir au final un produit à filtrer contaminé selon les spécifications d'un challenge bactérien standard.

4. Procédé de validation selon l'une des revendications précédentes, **caractérisé en ce qu'**il comporte au préalable une étape de validation du filtre stérilisant dans des conditions standards selon les règles de l'art i.e comportant un challenge bactérien et un test d'intégrité réalisés à température ambiante avec des solutions aqueuses.

5. Procédé de validation selon la revendication 4, **caractérisé en ce que** ladite étape de validation dans des conditions standards est suivie d'une étape de réalisation d'un test d'intégrité dans les conditions d'utilisation du filtre i. e avec le produit à filtrer et à la température du produit à filtrer, selon la méthodologie suivante :

   - on abaisse la température du filtre jusqu'à la température du produit à filtrer et on le maintient à cette température pendant toute la durée de ladite étape de validation dans des conditions standards,
   - on sature les pores du filtre avec le produit à filtrer,
   - on procède à l'injection d'un gaz propulseur qui reste à l'état gazeux à la température du produit à filtrer,
   - on augmente graduellement la pression du gaz propulseur jusqu'à obtenir l'expulsion du produit à filtrer des pores du filtre et obtenir ainsi une valeur de pression d'expulsion.

6. Procédé de validation selon la revendication 5 **caractérisé en ce que** l'on effectue une corrélation entre le test d'intégrité ainsi réalisé dans les conditions d'utilisation avec le test d'intégrité réalisé dans les conditions standards de façon à déterminer la valeur limite de pression, en tenant compte de la nature du produit, de la nature du gaz propulseur, et desdites conditions d'utilisation.

**7.** Procédé de validation selon la revendication 5 **caractérisé en ce que** l'on procède à l'observation même de l'allure de la courbe d'évolution de la pression afin de contrôler que le filtre n'est pas endommagé.

**8.** Procédé de validation selon l'une des revendications 5 à 7 **caractérisé en ce que** l'on effectue une corrélation entre ledit test d'intégrité réalisé dans les conditions d'utilisation et ledit test de challenge microbien ou particulaire réalisé avec le produit à filtrer.

**9.** Installation de validation d'un filtre stérilisant ou d'abattement microbien, en conditions d'utilisation, **caractérisée en ce qu'**elle comporte :

- des moyens pour abaisser la température du filtre jusqu'à la température du produit à filtrer et de maintenir le filtre à cette température pendant toute la durée de la procédure ;
- des moyens pour injecter dans le filtre une quantité déterminée du produit à filtrer, préalablement chargé en microorganismes ou en particules selon une concentration prédéfinie ;

lesdits moyens pour injecter dans le filtre une quantité déterminée du produit à filtrer, préalablement chargé en microorganismes ou en particules selon une concentration prédéfinie comprenant :

i) un réservoir intermédiaire destiné à stocker du produit à filtrer ;
j) un inoculum microbien ou particulaire en solution ;
k) des moyens d'introduction dudit inoculum microbien ou particulaire en solution dans ledit réservoir et d'évaporation dudit inoculum ainsi introduit.
l) des moyens d'abaissement de la température du réservoir jusqu'à la température du produit à filtrer et de maintien de la température du réservoir à cette température ;
m) des moyens d'introduction du produit à filtrer dans le réservoir ainsi contaminé après évaporation dudit inoculum ;

la concentration microbienne ou particulaire dudit inoculum en solution étant apte à permettre que le produit à filtrer obtenu en sortie du réservoir contienne une quantité de microorganismes ou de particules conforme aux spécifications recherchées.

# EP 1 508 362 A1

**Office européen des brevets**

## RAPPORT DE RECHERCHE EUROPEENNE

Numéro de la demande

EP 04 29 2042

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A,D | DE 33 42 440 A (MESSER GRIESHEIM GMBH) 5 juin 1985 (1985-06-05) * le document en entier * ----- | 1,9 | B01D65/10 G01N15/08 A61L2/28 |
| A | EP 0 569 754 A (JAGENBERG AG) 18 novembre 1993 (1993-11-18) * le document en entier * ----- | 1,9 | |
| A | DE 101 16 335 C (SARTORIUS GMBH) 17 octobre 2002 (2002-10-17) * revendications 1,5; figure 1 * ----- | 1,9 | |
| A | DE 101 51 270 A (SARTORIUS GMBH) 8 mai 2003 (2003-05-08) * revendications 1,5; figure 1 * ----- | 1,9 | |
| A | US 4 619 136 A (ORTIZ JOHN P) 28 octobre 1986 (1986-10-28) * le document en entier * ----- | 1,9 | |

**DOMAINES TECHNIQUES RECHERCHES (Int.Cl.7)**

B01D
G01N
A61L

Le présent rapport a été établi pour toutes les revendications

| Lieu de la recherche | Date d'achèvement de la recherche | Examinateur |
|---|---|---|
| Munich | 12 octobre 2004 | de Biasio, A |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

8

EP 1 508 362 A1

**ANNEXE AU RAPPORT DE RECHERCHE EUROPEENNE
RELATIF A LA DEMANDE DE BREVET EUROPEEN NO.**  EP 04 29 2042

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

12-10-2004

| Document brevet cité au rapport de recherche | | Date de publication | Membre(s) de la famille de brevet(s) | | Date de publication |
|---|---|---|---|---|---|
| DE 3342440 | A | 05-06-1985 | DE | 3342440 A1 | 05-06-1985 |
| EP 0569754 | A | 18-11-1993 | DE | 4215783 A1 | 18-11-1993 |
|  |  |  | DE | 59308561 D1 | 25-06-1998 |
|  |  |  | EP | 0569754 A1 | 18-11-1993 |
| DE 10116335 | C | 17-10-2002 | DE | 10116335 C1 | 17-10-2002 |
|  |  |  | WO | 02078823 A1 | 10-10-2002 |
|  |  |  | US | 2004129060 A1 | 08-07-2004 |
| DE 10151270 | A | 08-05-2003 | DE | 10151270 A1 | 08-05-2003 |
| US 4619136 | A | 28-10-1986 | AUCUN | | |

Pour tout renseignement concernant cette annexe : voir Journal Officiel de l'Office européen des brevets, No.12/82

9